# EUROPEAN PATENT SPECIFICATION

(11) **EP 3 370 628 B1**
(45) Date of publication and mention of the grant of the patent: **07.12.2022**
(21) Application number: 16794015.4
(22) Date of filing: 07.11.2016
(51) Int. Cl.: A61B 17/16

(54) **ROTARY CUTTER FOR PREPARING THE FEMUR BONE FOR A RESURFACING HIP IMPLANT**
ROTIERENDES SCHNEIDWERKZEUG ZUR PRÄPARATION DES FEMURKNOCHENS FÜR EIN OBERFLÄCHENERSATZ-HÜFTIMPLANTAT
COUTEAU ROTATIF POUR PRÉPARER LE FÉMUR POUR UN IMPLANT DE HANCHE DE RESURFAÇAGE

(30) Priority: 06.11.2015 GB 201519630
(43) Date of publication of application: 12.09.2018
(73) Proprietor: Embody Orthopaedic Limited, Exhibition Road London SW7 2PG (GB)
(72) Inventor: WOZENCROFT, Robert, Epsom, Surrey KT19 8PQ (GB)
(74) Representative: Mays, Julie
(86) International application number: PCT/GB2016/053478
(87) International publication number: WO 2017/077344

(56) References cited:
- WO-A1-2004/032767
- DE-A1-102014 203 456
- US-A1- 2011 184 419

## Description

### Background

During a hip resurfacing operation the head of the femur is retained and capped with a head implant with a spherical bearing of a similar size to the natural joint. The head of the femur is shaped with rotary cutters and sometimes a flat saw cut, so that the internal profile of the resurfacing head implant fits the femur bone precisely. In the case of cemented head implants the bone is machined slightly undersized so there is an even layer of bone cement between implant and bone. In the case of porous coated (cement free) resurfacing head implants, a small interference fit is required so that the head implant is a tight fit on the bone until bone ingrowth into the porous surface occurs to further strengthen fixation. If there is no interference or too little interference the head implant may not be stable enough initially and the implant could fail. If there is too much interference the head implant will be very difficult to fit. Therefore the rotary cutters must be precise enough to provide either a small clearance fit or the more critical small interference fit. Existing resurfacing head implants have a largely cylindrical bore with a flat end and either chamfered or dome sides' in- between. Typically the rotary cutters correspond to these shapes and include cylinder cutters, planar face cutters and chamfer cutters to shape the head of the femur in stages. However, some systems have a saw guide for making the flat planar cut and some have cutters to combine the shaping operations, such as combined cylinder and chamfer cutters or combined chamfer and planar face cutters. The cutters are guided in use by a central guide rod which is placed beforehand in the femur bone at the correct angle and orientation. Therefore rotary cutters include cutting end features, a bore which fits over a guide rod and a standard drive feature for attachment to a powered surgical drill. Some also include plastic attachments for collecting bone cuttings in use. US 2011/184419 relates to a patient specific acetabular guide and associated instruments and WO 2004/032767 relates to a single sized reamer for preparing the articulating surface of a bone.

Existing resurfacing instruments are reusable so they must be cleaned and sterilised before use and between each use. Cleaning can take several days, therefore if a hospital has only a small number of acetabular reamers this can reduce the rate at which patients can be treated as the surgeon must wait for the reamer to be returned from cleaning before the next patient can be treated. In general reusable instruments bring an increased risk of infection to the patient as there is a chance they will not be cleaned thoroughly enough or sterilised correctly. Instruments with bone cutting functions become heavily contaminated with bone and tissue debris in use and are particularly difficult to clean. Furthermore they are hazardous to staff involved with handling and cleaning due to the sharp cutting edges. In addition to the risks and difficulties of reprocessing reusable cutters, they also become blunt after several uses and cleaning cycles and need to be either replaced or sharpened. Rotary cutters are expensive to manufacture due to their complexity and the need for sharp cutting teeth which are typically formed in several stages of manufacture. Furthermore a set of cutters includes many size variants corresponding to the head implant size range, so as well as being expensive to manufacture, they take up a lot of space in the operating theatre. Reprocessing and maintenance cost are also high due to the difficulties discussed above.

### Statement of Invention

To overcome these difficulties, the present invention proposes a nest of rotary cutters as claimed in claim 1.

### Advantages and description

In one embodiment of the invention there is provided a nest of rotary cutters where each rotary cutter, is preferably designed for shaping the femur bone during a hip resurfacing operation, with a body comprising at least one portion comprising at least one cutting means, said cutter provided by a single metal component manufactured by additive manufacture wherein the body of the cutter comprises a substantially cylindrical region defining a distal part of the cutter and wherein the body of the cutter (9) further comprises a portion for fixing said cutter (9) to a holder (7), said fixing portion (18) defining a proximal part of the cutter (9), wherein the portion for fixing said cutter (9) to a holder comprises at least one leg (18) and wherein in the nest the respective portions for fixing each of said cutters (9) to the holder (7) are each of the same diameter so that a substantially cylindrical region is formed from said fixing portions when nested.

A number of additive manufacturing methods are known by the skilled person. One option is to use direct metal laser sintering (DMLS) and another option is electron beam melting (EBM). The cutter can be made from any suitable metal, such as steel (e.g. stainless steel), titanium or cobalt alloy (e.g. cobalt chrome).

The cutter typically will be defined by an approximately cylindrical body that has a distal end and a proximal end. For the sake of ease of description, the distal end is herein referred to as the end that will be positioned away from the surgeon in use. In other words, the end that will approach the bone first. This distal end comprises a substantially cylindrical region. The cylinder is defined by a wall of the cutter, and will be hollow internally. The diameter of the cylinder (measured either from external wall to external wall, or alternatively from internal wall to internal wall) will be chosen depending on the size of the bone that is to be cut. The void (hollow) within the cylindrical portion will accommodate the bone as the cutter is pushed over it.

Optionally the substantially cylindrical region can comprise one or more apertures. These may be useful for allowing bone fragments to be ejected from the cutter.

The body of the cutter also comprises a portion for fixing the cutter to a holder (described in more detail below). Again, for ease of reference herein, said fixing portion is said to define a proximal part of the cutter (i.e. it will be the part that is proximal to the holder/surgeon) .

Typically the cross section of the portion for fixing said cutter to a holder is of a smaller diameter than the distal cylindrical region. In preferred embodiments, there is a tapered region connecting said substantially cylindrical distal region to said proximal fixing region.

Depending on the intended use of the cutter, there may be a variety of arrangements of teeth placed at different positions on the cutter.

In order to execute a cylindrical cut on the femur bone, there will be cutting teeth positioned circumferentially at a distal end of the cylindrical region of the body. There can be any number of teeth, but typically will be more than one. For example, 1, 2, 3, 4, 5, 6, 7, 8. Preferably 8. These teeth are generally profiled such that as the rotary cutter is pushed over the bone, any part of the bone that extends beyond the internal diameter of the cylindrical portion is engaged by the teeth and cut away. Generally the teeth will have a slight angle to them in order to achieve efficient cutting.

Another arrangement of teeth that can be in addition to the teeth described above is where the cutting teeth are positioned on an inner surface of the body of the cutter.

If these teeth are axially inclined, for example positioned on at least a portion of the tapered region that connects the cylindrical distal portion with the proximal fixing portion, then in use they will be able to execute a chamfered cut on the femur bone as the cutter is pushed to engage the bone at the tapered region.

Again, these teeth can be profiled in any manner suitable to cause an even cut in the bone. As with any of the cutting teeth described herein, the teeth need not be formed of a single, flat cutting edge. Instead, they may comprise serrations or such like to affect efficient cutting. Moreover, in a rotary cutter where there are more than one inclined cutting tooth (e.g. 2, 3, 4, 5, 6, 7, 8, preferably 4), then any serrations present on each tooth may be offset in relation to one another such that there is no possibility that there will be a circumferential region on the bone that is not cut appropriated due to it falling in the path of a gap caused by a serration.

Where there is desired that the cutter is also able to cut the bone on its planar face, the cutter will also have teeth positioned internally in the cutter body such that they are positioned substantially perpendicular to the axis of the cutter. In some cases, the internal region of the cutter body where these cutting teeth are position may also form the start of the fixing portion, with the fixing means of the cutter extending proximally from this flat portion.

The portion of the cutter for fixing said cutter to a holder comprises at least one leg. Optionally 1, 2, 3, 4, 5 or 6. Preferably 4. These legs are designed to fit into corresponding receiving means on a holder. In some circumstances, the at least one leg further comprises a projection, said projection configured for a snap-fit connection with said holder where the holder has a complementary recess in order to accommodate the projection. It will be appreciated that the recess could be present in the at least one leg and the projection could be present in the holder.

In order to gain efficiencies of scale during the manufacturing process (thereby saving costs and materials), there is provided a nest of rotary cutters of decreasing/increasing diameters (of the substantially cylindrical distal region). The additive manufacturing process is able to leave a small gap between each cutter such that they are each removable from the nest. This also improves the ease of storage of the cutters.

In the nest, the respective portions for fixing each of said cutters to a holder are each of approximately the same diameter such that a substantially cylindrical region is formed from said fixing portions when nested. This can allow for standardised receiving portion sizes on holders to be made.

The holder of the present invention is designed to hold a rotary cutter of the invention at one end and to connect to a drive means at the other end, so that the drive means can rotate the cutter.

The holder comprises receiving means for receiving the fixing portion of said rotary cutter, said receiving means optionally comprising recesses complementary to any projections on the at least one leg of the fixing portion of said cutter.

Preferably the holder is manufactured by additive manufacture, and is preferably plastic (e.g. nylon).

The holder further comprises one or more apertures for collecting bone debris during cutting. The skilled person will be aware of the appropriate positions where these holes can be placed.

The holder may further incorporate a bore for engaging with a guide rod to guide the cutter in use, and/or a drive feature for attachment to a surgical power drill. The drive feature may be integrated with said holder, or may come as a separate part that is removably attachable to said holder. The drive feature may have a cross bar to transmit torque more evenly to the holder.

There is also described a computer-readable medium having computer-executable instructions adapted to cause a 3D printer to print a cutter and/or a holder as described herein.

There is also described a cutting system comprising a rotary cutter and a holder as described herein. The system may optionally come preassembled.

There is also described a method of shaping a femur bone during a hip resurfacing operation, said method comprising the use of a rotary cutter of the nest of the present invention, typically in combination with a holder as described herein.

In use, if the cutter has distal teeth and inclined teeth on the internal surface of the cutter body, then the rotary cutter is able to execute a cylindrical cut and chamfered cut on the femur bone in unison.

If the rotary cutter only has distal teeth, then it is able to execute a cylindrical cut on the femur bone.

If the rotary cutter only has inclined teeth on an internal surface, then it is able to execute a chamfered cut on the femur bone.

If the rotary cutter has distal teeth, inclined teeth on an internal surface, and flat teeth on an internal surface perpendicular to the axis, then it is able to execute a cylindrical cut, chamfered cut and planar face cut on the femur bone during one operation.

### Supplemental description and advantages

The advantage of additive manufacture is that the complex geometries of single or combined rotary cutters can be produced without the disadvantage of the many and complex manufacturing operations which are required with conventional manufacturing.

Preferably the rotary cutter is a combined cylinder and chamfer cutter although alternatively the following may be provided:
a) Separate cylinder cutter
b) Separate chamfer cutter
c) Combined chamfer and planar face cutter
d) Combined cylinder, chamfer and planar face cutter

Preferably, the rotary cutter will stop cutting on the planar flat cut already made at an earlier stage by a separate planar face cutter or saw cut. The invention provides the nesting several cutter sizes together within one another which is beneficial for cost effective manufacture via the AM process. Therefore many more cutters can be produced within the limited machine build capacity than if they were built individually (approximately four to five times as many). It also provides space saving benefits for pre-assembled parts and space saving in the operating theatre if complete sets of cutters are provided for self-assembly. Furthermore, it is proposed that any of the alternative cutter options listed above (a-d) will be nested together in the same way for these benefits. The present invention as a single use nest of cutters will preferably be supplied sterile packed and will be disposed of rather than reprocessed after use. The cutting features will be sufficiently accurate and sharp and as they are not reused will not go blunt like conventional reusable cutters. Preferably, the cutters will be preassembled into a plastic holder which incorporates an appropriately sized bore for following the guide rod, apertures for collecting bone debris during cutting and a standard drive for attachment to a surgical power drill. Alternatively, the metal cutters may be provided separately or in a set for self-assembly with the holder by the operating theatre staff during a resurfacing operation. The rotary cutter has a multitude of cutting teeth for smooth cutting of bone, preferably but not limited to eight cutting teeth for the cylindrical cut and four cutting teeth for the chamfer cut.

In manufacture it may be desirable to improve the cutting accuracy (e.g. size and roundness) in particular of the cylinder cutting section of the cutter, so that the cut cylindrical portion of the femoral head is more accurately machined for the slight interference fit with the implant. It may therefore be desirable to grind the bore which is a very accurate machining process capable of producing a tolerance of plus or minus 50 microns or less. Furthermore it may be desirable to improve the cutting effectiveness of all cutting edges, so the design allows for access to sharpen all cutting edges with a suitable tool (for example a manual file, or power file or small grinding wheel.

### Introduction to drawings

An example of the invention will now be described by referencing to the accompanying drawings:
Figure 1 is an exploded view of the pre-prepared femur bone and resurfacing head implant.
Figure 2 is a cross sectioned view of the resurfacing head implant fitted to the femur bone.
Figure 3 is the fully assembled rotary cutter.
Figure 4 is an exploded view of the rotary cutter.
Figure 5 is an orthographic view of the rotary cutter.
Figure 6 is a side view of the rotary cutter.
Figure 7 is a cross section of figure 6.
Figure 8 is a close up details of a portion of figure 7.
Figure 9 shows the rotary cutter as it is about to cut the femur bone.
Figure 10 shows the rotary cutter after it has cut the femur bone.
Figure 11 is the metal cutter part of the rotary cutter.
Figure 12 shows several metal cutter parts nested together as in manufacture.
Figure 13 is an exploded view of the nested cutters in figure 12

### Description with reference to drawings

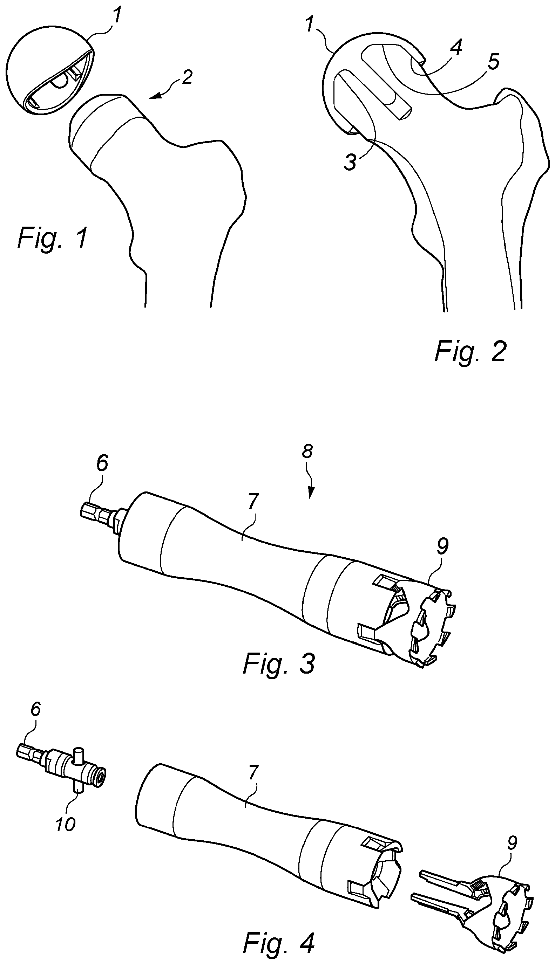

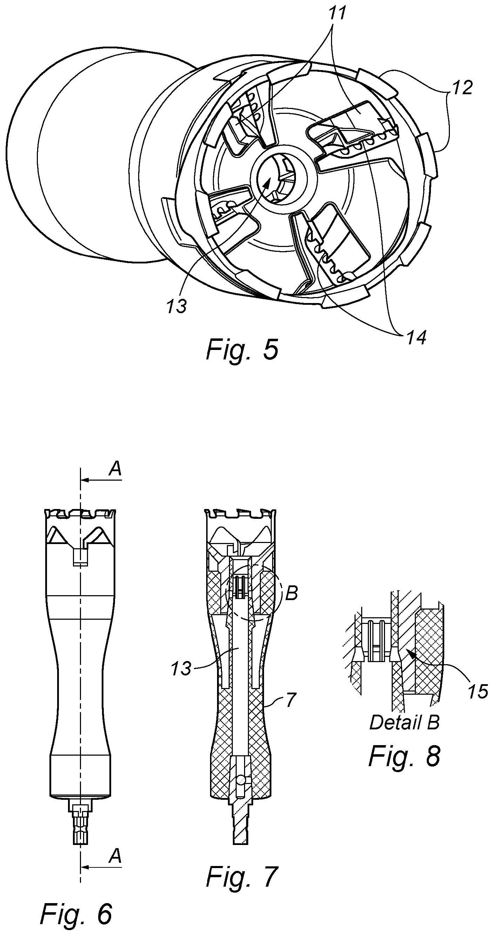

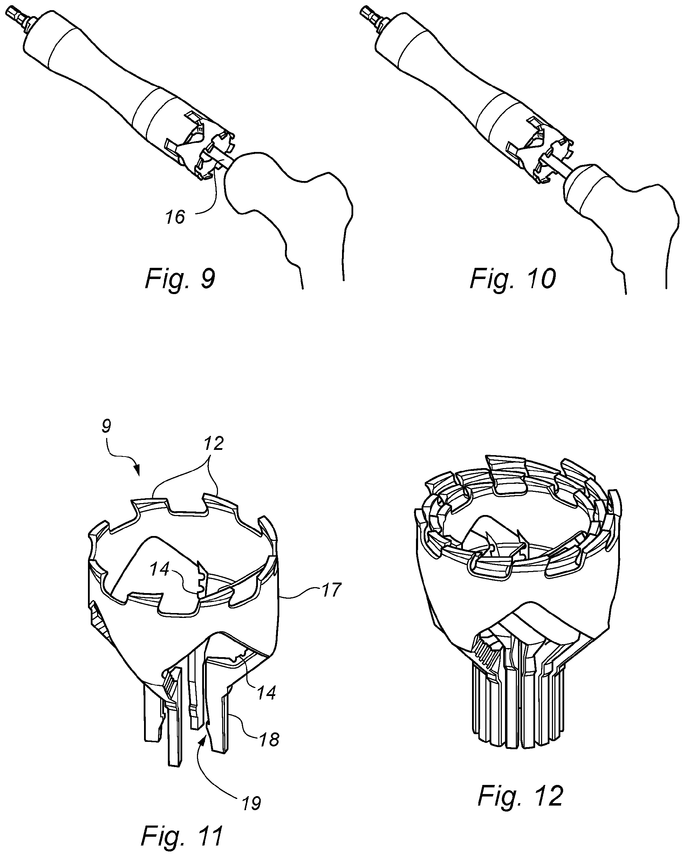

As described above a resurfacing hip operation involves shaping the head of the femur (2) for the precise fitting of a resurfacing head implant (1) as shown in figures 1 & 2. In figure 2 it can be seen that the internal profile of the cross sectioned resurfacing head implant has a cylindrical bore (4) with a flat planar end portion (3) with a chamfered portion (5) in between and that the head of the femur bone (2) is shaped to match. The rotary cutter (8) as depicted in figures 3-13 will make both the cylindrical and chamfered cuts on the femur bone. As shown in figures 3 & 4, it consists of three parts, a metal cutter (9), a holder (7) preferably made from plastic and a drive (6) preferably made from metal for attachment to a surgical power drill (not shown). The drive part is press fitted into the holder and may incorporate a separate pin (10) for transferring torsional forces to the holder. Figure 5 shows cutting teeth (12) for making the cylindrical cut and cutting teeth (14) for making the chamfer cut. Also shown in figure 5 are apertures adjacent to the chamfer cutting teeth (11) for collecting bone debris during cutting and a bore (13) for following a guide rod (16) shown in later figures 9 & 10. In figure 7 the bore (13) is seen to extend into most of the length of the holder (7). Figure 8 shows a snap fit feature (15) for fixing the metal cutter (9) into the holder (7). In use the rotary cutter is assembled in a surgical power drill (not shown) and advanced over a guide rod (16) which is prepositioned in the femur bone (2) as shown in figures 9 & 10. It is rotated at a low speed to make a controlled cut (figure 9 shows before and figure 10 after the bone cut).

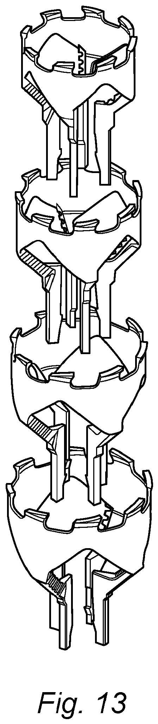

The separated metal cutter (9) is shown in figure 11 incorporating cutting teeth (12) for the cylinder cut and cutting teeth (14) for the chamfer cut, a cylindrical body (17) and legs (18) for insertion into the holder (7). Male snap fit features (19) provide fixation with the holder. Figure 12 shown how several sizes of cutters (in this case four) are nested together both for the AM manufacturing process and for storage. In figure 13 the four sizes of nested cutters are exploded apart for clarity.

## Claims

1. A nest of rotary cutters wherein two or more different sized cutters are nested together within one another during manufacture and/or for storage, each cutter (9) with a body comprising at least one portion comprising at least one cutting means (12,14), said cutter (9) provided by a single metal component manufactured by additive manufacture wherein the body of the cutter comprises a substantially cylindrical region (17) defining a distal part of the cutter (9):
wherein the body of the cutter (9) further comprises a portion for fixing said cutter (9) to a holder (7), said fixing portion (18) defining a proximal part of the cutter (9),
wherein the portion for fixing said cutter (9) to a holder comprises at least one leg (18) and
wherein in the nest the respective portions for fixing each of said cutters (9) to the holder (7) are each of the same diameter so that a substantially cylindrical region is formed from said fixing portions when nested.

2. A nest of rotary cutters of claim 1, in which each cutter (9) is single use and optionally where said substantially distal cylindrical region (17) comprises one or more apertures (11).

3. A nest of rotary cutters of claim 1, wherein:
(i) the cross section of the proximal fixing portion for fixing said cutters (9) to a holder (7) is of a smaller diameter than the distal cylindrical region (17); or
(ii) there is a tapered region connecting said substantially cylindrical distal region (17) of each cutter to said proximal fixing region.

4. A nest of rotary cutters according to any preceding claim, wherein said at least one cutting means comprises cutting teeth (12) positioned circumferentially at a distal end of the body.

5. A nest of rotary cutters according to any preceding claim, wherein said at least one cutting means comprises cutting teeth (14) positioned on an inner surface of the body of the cutter.

6. A nest of rotary cutters of claim 5, wherein the configuration of said teeth (14) is such that they are axially inclined, wherein said teeth (14) are positioned on at least a portion of the tapered region.

7. A nest of rotary cutters of claim 5, wherein the configuration of said teeth is such that they are positioned substantially perpendicular to the axis of the cutter.

8. A nest of rotary cutters of claim 1 wherein said at least one leg (18) further comprises a projection (19), said projection (19) configured for a snap fit connection with said holder (7).

9. A nest of rotary cutters of any preceding claim, wherein said cutter is metal, such as steel (e.g. stainless steel), titanium, or cobalt alloy (e.g. cobalt chrome).

10. A nest of rotary cutters of any preceding claim wherein the portion for fixing said cutter (9) to a holder comprises from 2 to 6 legs (18).

11. A nest of rotary cutters of any preceding claim wherein the portion for fixing said cutter (9) to the holder (7) comprises 4 legs (18).

12. A nest of rotary cutters of any preceding claim,
wherein the nest has four rotary cutters.

## Patentansprüche

1. Satz rotierender Schneidwerkzeuge, wobei zwei oder mehr unterschiedlich große Schneidwerkzeuge während der Herstellung und/oder zur Lagerung ineinander geschachtelt werden, wobei jedes Schneidwerkzeug (9) einen Körper aufweist, der mindestens einen Abschnitt umfasst, der mindestens ein Schneidmittel (12, 14) umfasst, wobei das Schneidwerkzeug (9) durch eine einzelne Metallkomponente bereitgestellt wird, die durch additive Fertigung hergestellt ist, wobei der Körper des Schneidwerkzeugs einen im Wesentlichen zylindrischen Bereich (17) umfasst, der einen distalen Teil des Schneidwerkzeugs (9) definiert:
wobei der Körper des Schneidwerkzeugs (9) ferner einen Abschnitt zum Befestigen des Schneidwerkzeugs (9) an einem Halter (7) umfasst, wobei der Befestigungsabschnitt (18) einen proximalen Teil des Schneidwerkzeugs (9) definiert,
wobei der Abschnitt zum Befestigen des Schneidwerkzeugs (9) an einem Halter mindestens ein Bein (18) umfasst und
wobei in dem Satz die jeweiligen Abschnitte zum Befestigen jedes der Schneidwerkzeuge (9) an dem Halter (7) jeweils den gleichen Durchmesser aufweisen, sodass ein im Wesentlichen zylindrischer Bereich aus den Befestigungsabschnitten gebildet wird, wenn sie ineinander geschachtelt sind.

2. Satz rotierender Schneidwerkzeuge nach Anspruch 1, bei dem jedes Schneidwerkzeug (9) zum einmaligen Gebrauch bestimmt ist und bei dem optional der im Wesentlichen distale zylindrische Bereich (17) eine oder mehrere Öffnungen (11) umfasst.

3. Satz rotierender Schneidwerkzeuge nach Anspruch 1, wobei:
(i) der Querschnitt des proximalen Befestigungsabschnitts zum Befestigen der Schneidwerkzeuge (9) an einem Halter (7) einen kleineren Durchmesser als der distale zylindrische Bereich (17) hat; oder
(ii) es einen sich verjüngenden Bereich gibt, der den im Wesentlichen zylindrischen distalen Bereich (17) jedes Schneidwerkzeugs mit dem proximalen Befestigungsbereich verbindet.

4. Satz rotierender Schneidwerkzeuge nach einem vorhergehenden Anspruch, wobei das mindestens eine Schneidmittel Schneidzähne (12) umfasst, die in Umfangsrichtung an einem distalen Ende des Körpers positioniert sind.

5. Satz rotierender Schneidwerkzeuge nach einem vorhergehenden Anspruch, wobei das mindestens eine Schneidmittel Schneidzähne (14) umfasst, die an einer inneren Oberfläche des Körpers des Schneidwerkzeugs positioniert sind.

6. Satz rotierender Schneidwerkzeuge nach Anspruch 5, wobei die Konfiguration der Zähne (14) derart ist, dass sie axial geneigt sind, wobei die Zähne (14) auf mindestens einem Abschnitt des sich verjüngenden Bereichs positioniert sind.

7. Satz rotierender Schneidwerkzeuge nach Anspruch 5, wobei die Konfiguration der Zähne derart ist, dass sie im Wesentlichen senkrecht zur Achse des Schneidwerkzeugs positioniert sind.

8. Satz rotierender Schneidwerkzeuge nach Anspruch 1, wobei das mindestens eine Bein (18) ferner einen Vorsprung (19) umfasst, wobei der Vorsprung (19) für eine Schnappverbindung mit dem Halter (7) konfiguriert ist.

9. Satz rotierender Schneidwerkzeuge nach einem vorhergehenden Anspruch, wobei das Schneidwerkzeug aus Metall besteht, wie etwa Stahl (z. B. rostfreier Stahl), Titan oder eine Kobaltlegierung (z. B. Kobalt-Chrom).

10. Satz rotierender Schneidwerkzeuge nach einem vorhergehenden Anspruch, wobei der Abschnitt zum Befestigen des Schneidwerkzeugs (9) an einem Halter 2 bis 6 Beine (18) umfasst.

11. Satz rotierender Schneidwerkzeuge nach einem vorhergehenden Anspruch, wobei der Abschnitt zum Befestigen des Schneidwerkzeugs (9) an dem Halter (7) 4 Beine (18) umfasst.

12. Satz rotierender Schneidwerkzeuge nach einem vorhergehenden Anspruch, wobei der Satz vier rotierende Schneidwerkzeuge aufweist.

## Revendications

1. Faisceau de couteaux rotatifs, deux, ou plus, couteaux de tailles différentes étant imbriqués l'un dans l'autre durant la fabrication et/ou pour le stockage, chaque couteau (9) avec un corps comprenant au moins une partie comprenant au moins un moyen de coupe (12, 14), ledit couteau (9) étant fourni par un composant métallique unique fabriqué par fabrication additive, ledit corps du couteau comprenant une zone sensiblement cylindrique (17) définissant une partie distale du couteau (9) :
ledit corps du couteau (9) comprenant en outre une partie destinée à fixer ledit couteau (9) à un support (7), ladite partie de fixation (18) définissant une partie proximale du couteau (9),
ladite partie destinée à fixer ledit couteau (9) à un support comprenant au moins une patte (18) et
dans le faisceau, lesdites parties respectives destinées à fixer chacune desdits couteaux (9) au support (7) étant chacune du même diamètre afin qu'une zone sensiblement cylindrique soit formée à partir desdites parties de fixation lorsqu'elles sont imbriquées.

2. Faisceau de couteaux rotatifs selon la revendication 1, chaque couteau (9) étant à usage unique et éventuellement où ladite zone cylindrique sensiblement distale (17) comprend une ou plusieurs ouvertures (11).

3. Faisceau de couteaux rotatifs selon la revendication 1,
(i) ladite section transversale de la partie de fixation proximale destinée à fixer lesdits couteaux (9) à un support (7) étant d'un diamètre plus petit que la zone cylindrique distale (17) ; ou
(ii) une zone conique reliant ladite zone distale sensiblement cylindrique (17) de chaque couteau à ladite zone de fixation proximale étant présente.

4. Faisceau de couteaux rotatifs selon une quelconque revendication précédente, ledit au moins un moyen de coupe comprenant des dents de coupe (12) positionnées circonférentiellement au niveau d'une extrémité distale du corps.

5. Faisceau de couteaux rotatifs selon une quelconque revendication précédente, ledit au moins un moyen de coupe comprenant des dents de coupe (14) positionnées sur une surface interne du corps du couteau.

6. Faisceau de couteaux rotatifs selon la revendication 5, ladite configuration desdites dents (14) étant de sorte qu'elles soient inclinées axialement, lesdites dents (14) étant positionnées sur au moins une partie de la zone conique.

7. Faisceau de couteaux rotatifs selon la revendication 5, ladite configuration desdites dents étant de sorte qu'elles soient positionnées sensiblement perpendiculaires à l'axe du couteau.

8. Faisceau de couteaux rotatifs selon la revendication 1, ladite au moins une patte (18) comprenant en outre une saillie (19), ladite saillie (19) étant conçue pour un raccordement par encliquetage avec ledit support (7).

9. Faisceau de couteaux rotatifs selon une quelconque revendication précédente, ledit couteau étant en métal, tel que de l'acier (par exemple de l'acier inoxydable), du titane ou un alliage de cobalt (par exemple du chrome-cobalt).

10. Faisceau de couteaux rotatifs selon une quelconque revendication précédente, ladite partie destinée à fixer ledit couteau (9) à un support comprenant de 2 à 6 pattes (18).

11. Faisceau de couteaux rotatifs selon une quelconque revendication précédente, ladite partie destinée à fixer ledit couteau (9) au support (7) comprenant 4 pattes (18).

12. Faisceau de couteaux rotatifs selon une quelconque revendication précédente, ledit faisceau possédant quatre couteaux rotatifs.
